# EUROPEAN PATENT SPECIFICATION

(11) **EP 1 340 480 B1**
(45) Date of publication and mention of the grant of the patent: **03.02.2010**
(21) Application number: 03075516.9
(22) Date of filing: 24.02.2003
(51) Int. Cl.: A61F 13/15

(54) **Transpirant biodegradable film and absorbent sanitary articles incorporating it**
Gasdurchlässige biologischabbaubare Folie und absorbierende Hygieneartikel, die diese enthalten
Film biodégradable perméable aux gaz et articles hygièniques absorbants le contenant

(30) Priority: 27.02.2002 IT MI20020387
(43) Date of publication of application: 03.09.2003
(73) Proprietor: Corman S.p.A., 20084 Lacchiarella MI (IT)
(72) Inventor: Oldani Osvaldo, 34013 Duino Aurisina (Trieste) (IT); Tesolin Laura, 33170 Pordenone (IT)
(74) Representative: Cicogna, Franco

(56) References cited:
- WO-A-93/00399
- WO-A-93/03098
- WO-A-97/29909
- WO-A-98/43810
- AU-B- 741 001

## Description

The present invention relates to a transpirant biodegradable film and biodegradable absorbent sanitary articles incorporating it.

In particular, the present invention relates to a transpirant film made of a biodegradable plastic material and absorbent sanitary articles of the disposable type which incorporate it as an external wrapping for containing body fluids and exudates.

It is known that a wide variety and numerous types of sanitary articles for the absorption of body fluids are available on the market.

The disposable use of these absorbent articles together with their progressively increasing diffusion has created, in the last few years, problems relating to their disposal.

The absorbent sanitary articles currently on the market have a biodegradability of about 40% of the components as they are mainly made of synthetic plastic materials.

For example, children's nappies incorporate an external film made of non-biodegradable plastic on which non-woven fabrics normally made of polypropylene, polyester, adhesive polypropylene or polyester tapes, polypropylene elastic bands, polyurethane elastic threads, hotmelt glues and super-absorbent polymers made of sodium polyacrylate mixed with natural cellulose fibres, are supported. A nappy of this type, having an average weight of 50 g normally incorporates cellulose as sole biodegradable material, in a weight of about 20 g. Attempts made at substituting the plastic materials currently used in nappies with other biodegradable materials have had limited success due to the difficulty in finding a biodegradable product which is also impermeable to body fluids.

WO 93/03098 discloses a film made of biodegradable thermoplastic material with a microporous structure allowing transpiration while maintaining impermeability.

WO 97/29909 discloses a method for making a cloth-like microporous laminate of a nonwoven fibrous web and thermoplastic film that provide air and moisture vapour permeabilities with liquid barrier properties.

One of the objectives of the present invention is therefore to provide a transpirant biodegradable film having an impermeability to liquids which makes it suitable for use as an external supporting layer in absorbent articles for the human body.

Another objective of the present invention consists in providing a sanitary absorbent article of the disposable type having an increased biodegradability.

Yet another objective consists in providing a nappy of the disposable type which combines, in a single article, a high wearing comfort with improved biodegradability characteristics without jeopardizing the child's physiological skin perspiration.

In view of these objectives and others which will appear evident hereafter, a first aspect of the present invention relates to a biodegradable sanitary absorbent article comprising a film made of biodegradable thermoplastic material, particularly suitable for being used as a layer for containing body fluids in absorbent articles, characterized in that it is microporous due to the presence of a series of micropores which penetrate its thickness allowing transpiration, it is impermeable to liquids and has a gram-surface ratio within the range of 18-27.5 g/m² and has transpirability values ranging from about 1300 to 1700 g/sm/24h.

It has been found that with these specific gram-surface ratio values of the film, the skin perspiration of water vapour and impermeability to fluids are optimized without jeopardizing the mechanical resistance properties. It has been found, for example, that with gram-surface ratio values within the range of 24 and 25.5 g/m², transpirability values ranging from about 1300 to 1700 g/sm/24h are obtained, measured with the Mokon method, due to the presence of micro-holes (microporosity) which penetrate the thickness of the film. Particularly suitable transpirability values of the bioplastic film range from 1550 to 1670 and preferably from 1600 to 1650 g/sm/24h.

Biodegradable thermoplastic materials which are particularly suitable for producing the film of the invention consist of bio-plastics such as Mater-Bi.

These thermoplastic materials, deriving from natural raw materials such as maize starch, maintain the same characteristics of use in absorbent articles made of plastic materials traditionally adopted, even though they are essentially biodegradable. The selected gram-surface ratio values of the bio-plastic film make them transpirant and impermeable to liquids thus allowing the retention of body liquids without jeopardizing the physiological conditions of skin perspiration, when the film is applied close to a person's skin.

Furthermore, the film of the invention combines, in a single product, hydrophobic characteristics and transpirability to vapour, with a good mechanical resistance which make it particularly suitable for use as an external wrapping for containing fluids in sanitary absorbent articles. In addition, the reduced gram-surface ratio of the film of the invention creates advantages relating to cost, transportation and efficiency of the production line of absorbent articles.

With the same diameter, in fact, the film bobbins with a reduced thickness have a greater length allowing, with the same volume, the transportation of a higher number of linear metres.

Furthermore, in the production line of absorbent articles such as nappies, the greater length of film allows a reduction in the number of changes in material which coincide with waste product. This leads to a considerable increase in process efficiency and yield.

The film of the invention conveniently has mechanical resistance properties which make it suitable for the required uses and which are summarized in the following Table.

| Mechanical characteristics | Measurement Unit | Method | Range of values | Preferred range |
|---|---|---|---|---|
| MD elongation 5% | N/m | ASTM D882-95 | 0.01016-0.02159 | 0.01397-0.01651 |
| MD elongation 10% | N/m | ASTM D882-95 | 0.0508-0.1778 | 0.1016-0.127 |
| MD Ultimate tensile strength | N/m | ASTM D882-95 | 0.5334-0.889 | 0.6858-0.7366 |
| MD ultimate elongation | N/m | ASTM D882-95 | 3.302-9.144 | 5.334-6.35 |
| CD elongation 5% | N/m | ASTM D882-95 | 0.02032-0.09144 | 0.0508-0.06096 |
| CD elongation 10% | N/m | ASTM D882-95 | 0.04318-0.11938 | 0.0762-0.08636 |
| CD Ultimate tensile strength | N/m | ASTM D882-95 | 0.1778-0.4064 | 0.2794-0.3048 |
| CD elongation | N/m | ASTM D882-95 | 12.7-22.86 | 16.51-19.05 |
| Friction coefficient | | ASTM D1894-94 | 0.01016-0.02159 | 0.01524-0.01651 |

The MD and CD data refer to the stretching values of the materials in the machine direction and transversal to the machine, respectively. The elongation data in the machine direction (MD) indicated above show that although the bioplastic film of the invention does not have high stretch properties, it has a low breakage susceptibility as the force necessary for breaking it ranges from 21 to 35 Newton.

The film of the invention can be produced by the extrusion of a biodegradable thermoplastic material. The extrusion is conveniently effected with the bubble blowing technique followed by coupling of the opposite surfaces of the thermoplastic bubble produced. The blowing phase of the film is conveniently carried out at about 2-4 atmospheres.

The micropores of the film of the invention are conveniently formed during the extrusion process, in particular in bubble extrusion following by coupling by pressing the bubble formed.

Alternatively, the micropores of the film can be produced with conventional microperforation techniques, for example of the type used for producing the transpirant membranes adopted in the clothing industry such as Gore-Tex.

The film of the invention is specifically suitable for use inside a sanitary absorbent article as an external transpirant layer for containing body liquids and exudates.

Another aspect of the present invention relates to a sanitary absorbent article comprising a film made of biodegradable thermoplastic material, as described above.

The article according to the invention conveniently comprises at least one internal layer permeable to liquids to be applied close to the pubic region of the person of interest, an external layer made of biodegradable thermoplastic material of the type previously described and an absorbing layer situated between said two layers, suitable for absorbing liquids and exudates of the human body.

The permeable internal layer and the external bioplastic layer are preferably firmly coupled by means of a glue of the hot melt type.

According to an embodiment, the absorbent article of the invention also has a barrier system suitable for substantially limiting the leakage of body exudates and means for its adherence to the body once applied.

The term sanitary absorbent article refers to articles which, when positioned in contact with or close to the pubic-sacral region of a person, absorb and withhold various body fluids or exudates released by the body. These articles are generally conceived for being thrown away after single use. An article of this kind can be worn by small children or incontinent adults, after being positioned and fixed between the user's legs. The term sanitary absorbent article consequently comprises children's nappies and protective towels for incontinent adults.

The characteristics and advantages of a sanitary absorbent article according to the present invention will appear more evident from the following illustrative but non-limiting description, referring to the enclosed schematic drawings in which:
- figure 1 illustrates a structure of an embodiment of a nappy according to the invention destined for babies or small children;
- figure 2 shows an exploded view of an embodiment of a nappy according to the invention.

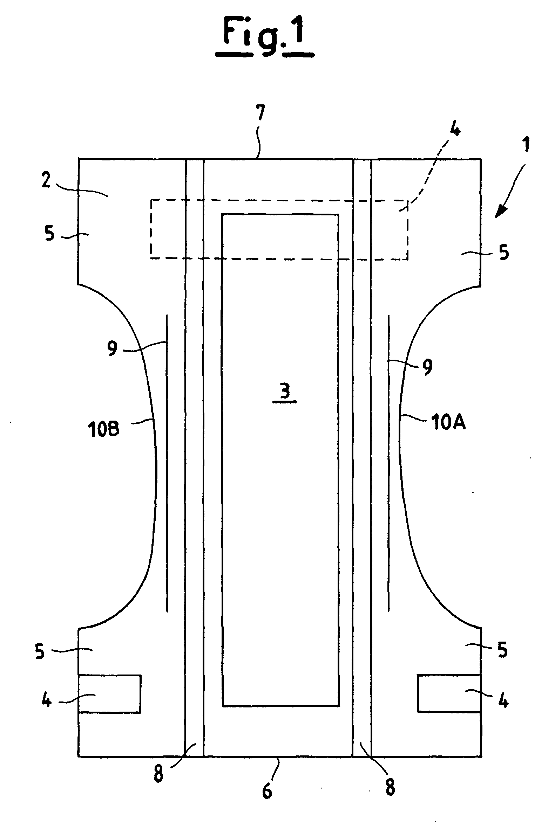

With reference to the enclosed figures, figure 1 illustrates a nappy, generally indicated with number 1, completely opened, before being worn by the user.

The nappy 1 comprises an upper film 2 permeable to liquids, an internal absorbing layer 3, a closing system 4 conveniently positioned on flaps 5 situated close to the end or lower cut 6 and close to the end or upper cut 7, fecal barriers 8 conveniently of the elasticized type and elasticized elements 9 situated close to the leg openings 10A, 10B.

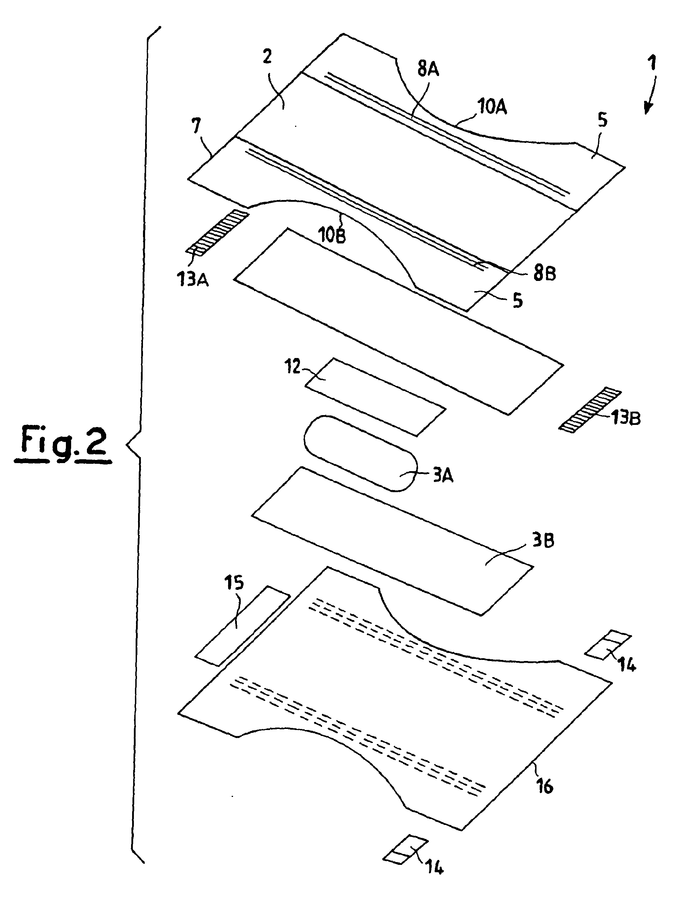

With reference to the enclosed figure 2, it can be observed that the upper permeable layer 2 of the nappy 1, has almost the same dimensions as the external lower layer 16 so as to create an exact overlapping of the two external layers of the nappy 1.

The perimeter of the nappy 1 is extended and is bordered from the upper end 7 to the lower end 6, by the side flaps 5 and by the two leg openings 10A and 10B.

The upper layer 2 of the nappy is made of a film permeable to body fluids which remains for the most part in contact with the user's skin. The side flaps 5 are the portions close to the upper end 7 and lower end 6 which overlap when the nappy 1 is being worn by the user and consequently when the nappy 1 is attached to the user's sides.

The internal absorbing layer 3, positioned between the upper layer 2 and biodegradable lower layer 16 of the invention, can have various shapes and dimensions proportional to the size of the nappy. This absorbing layer 3, which is suitable for absorbing and withholding body liquids and certain exudates, can, for example, be thin and pressed or bulky and conformable. It can be made of a variety of materials capable of withholding and absorbing urine commonly used in disposable absorbent articles. Examples of these absorbing materials comprise cellulose fluff, super-absorbent polymers, gelifying absorbing material, cotton or equivalent absorbing materials. The concentration and weight of the absorbing material varies from size to size to ensure that the expected quantity of liquids or exudates for the specific size is absorbed and withheld. The absorbing layer 3 can include a single or double layer. In particular, the enclosed Figure 2 illustrates a nappy 1 with a double absorbing layer: the upper absorbing layer 3A has the function of rapidly collecting the body liquids. Said upper absorbing layer 3A generally comprises fibres of hydrophilic material, without or with limited quantities of gelifying absorbent material.

This absorbing layer 3A is normally positioned in the area where there is the greatest necessity, which corresponds to the central-front area of the nappy, between the upper cut end 7 and the lower cut end 6. The lower absorbing layer 3B has the function of absorbing and withholding the liquids filtered by the upper layer 3A. The lower layer 3B, which generally has greater dimensions than the upper absorbing layer 3A, conveniently extends for almost the whole length of the nappy 1, from the upper end 7 to the lower end 6, compatibly with the possibility of sealing the nappy near the end. The absorbing layer can be positioned in continuous along the length of the nappy or intermittently so as to create areas with a greater concentration of absorbing material.

A thin collecting layer 12, also made of natural or synthetic transpirant material, and a lining layer 11 made of transpirant fabric, can conveniently be superimposed on said absorbing layers 3A and 3B.

As the upper layer 2 of the nappy 1 is in close contact with the user's skin, it must absorb the waste body liquids and is made of materials permeable to liquids, which are soft and non-irritating to allow their rapid passage to the underlying absorbing layer(s) 3. The upper filtering layer 2 can be made of natural fibres such as cellulose, cotton, viscose and other similar materials, synthetic fibres such as polypropylene, polyester and other similar products or a mixture of the two types of fibres.

The upper transpirant layer 2 advantageously comprises at least one hydrophobic portion which forms a barrier for feces in order to effectively prevent their leakage from the nappy. For example, said hydrophobic portion comprises two strips or barriers 8A and 8B made of hydrophobic material arranged longitudinally to the nappy 1, each close to the leg openings 10A and 10B of the nappy 1. These fecal barriers 8A, 8B conveniently comprise elastic elements, generally elastic filaments made of natural rubber or synthetic elastic materials such as polyurethane, suitable for the adherence of the nappy 1 to the user's legs and preventing the leakage of feces or liquids. According to another embodiment, said upper layer 2 of the nappy 1 comprises a hydrophilic portion which extends to the central part only so that it is situated above the absorbing layer 3. For example, said hydrophilic portion substantially extends longitudinally along the whole length of the upper surface 2 of the nappy and is bordered at the side by the presence of elastic barriers 8. The hydrophobic portion in this case extends along the side flaps and close to the leg openings 10A and 10B.

The nappy 1 of the invention can also be provided with elasticizing elements 9A and 9B situated close to the leg openings 10A and 10B respectively. These elastic elements 9A and 9B, advantageously comprise a series of stretched elastic threads capable of gripping the user's legs when the nappy 1 is applied. The nappy 1 can also comprise elastic bands 13A and B positioned near the ends 6 and 7 of the nappy 1 to ensure a firmer grip when applied to a person.

The outer layer 16 of the nappy 1 comprises a film made of biodegradable thermoplastic material, of the type described above.

This biodegradable film, is advantageously made of a bioplastic material such as Mater-Bi, is impermeable to body liquids and exudates thus preventing the exudates absorbed by the central absorbing layer 3 from fouling linen, sheets, clothes or other articles which come in contact with the nappy. As said outer layer 3 is permeable to the water vapour mainly formed in the absorbing layer 3, it prevents humidity from accumulating in the nappy 1 and irritating the skin or jeopardizing the physiological functioning of skin perspiration also in newly-born babies. In addition, the high biodegradability of the lower layer 16 considerably limits disposal problems linked to the use of disposable nappies.

The nappies of the invention have a more favourable environmental impact with respect to traditional nappies, as they do not significantly contribute to the impoverishment of natural substances.

Furthermore, the use of bioplastics for their production also allows a reduction in the degradation times of the cellulose absorbing component inside, thus creating a synergy in the biodegradability of the article.

According to an embodiment of the invention, said lower layer 16 of the nappy 1 is made opaque by the addition of suitable biodegradable pigments of a natural origin or with Mater Batch which make it appear like an item of clothing.

## Claims

1. A biodegradable sanitary absorbent article comprising a biodegradable film made of a biodegradable thermoplastic material, suitable as a transpirant layer for containing body fluids, **characterized in that** said film is microporous due to the presence of a series of micropores which pass through it allowing transpiration, it is impermeable to liquids, has a gram-surface ratio ranging from 18 to 27.5 g/m² and transpirability values ranging from about 1300 to 1700 g/sm/24h.

2. The article according to claim 1, **characterized in that** said film has a gram-surface ratio ranging from 22 to 26 g/m².

3. The article according to claim 1 or 2, **characterized in that** said gram-surface ratio ranges from 24 to 25.5 g/m².

4. The article according to any of the previous claims 1-3, **characterized in that** said film has an MD ultimate tensile strength ranging from 27 to 29 N/Inch and an MD ultimate elongation ranging from 210 to 250 N/inch.

5. The article according to any of the previous claims 1-4, **characterized in that** said film has a CD ultimate tensile strength ranging from 11 to 12 N/Inch and a CD ultimate elongation ranging from 650 to 750 N/Inch.

6. The article according to any of the previous claims 1-5, **characterized in that** said biodegradable thermoplastic material is Mater-Bi.

7. The article according to any of the previous claims 1-6, **characterized in that** it is opaque due to the presence of biodegradable natural pigments which give it an aesthetic appearance similar to an item of clothing. newly-born babies.

8. The article according to claim 6 or 7, **characterized in that** it also comprises a barrier system for substantially limiting the leakage of feces and liquids when worn.

9. The article according to any of the claims 6-8, **characterized in that** it also comprises a closing system positioned on outer flaps, elasticized fecal barriers close to two side leg openings.

10. The article according to any of the claims 6-9, **characterized in that** said central absorbing layer comprises a biodegradable absorbing material.

11. The article according to claim 10, **characterized in that** said biodegradable absorbing material comprises cellulose fluff, cotton, natural absorbing fibres and their mixtures.

12. The article according to any of the claims 6-11, **characterized in that** it also comprises one or more layers permeable to liquids arranged above said central absorbing layer.

13. The article according to any of the claims 6-12, **characterized in that** elastic threads are applied on the internal surface of said outer layer and close to the leg openings to prevent the leakage of body liquids.

14. The article according to any of the claims 6-13, **characterized in that** said outer layer impermeable to liquids comprises biodegradable natural pigments or Mater Batch to give said articles an appearance similar to an item of clothing.

15. The article according to any of claims 1-14 **characterised in that** it is a nappy.

## Patentansprüche

1. Biologisch abbaubarer Hygieneabsorptionsartikel mit einer biologisch abbaubaren Folie, die aus einem biologisch abbaubaren thermoplastischen Material hergestellt ist und als Transpirationsschicht zur Aufnahme von Körperfluiden geeignet ist, **dadurch gekennzeichnet, dass** die Folie aufgrund des Vorhandenseins einer Reihe von Mikroporen, die durch diese hindurch gehen, mikroporös ist, wodurch Transpiration ermöglicht wird, für Flüssigkeiten undurchlässig ist, ein Gramm-Oberflächen-Verhältnis aufweist, das in einem Bereich von 18 bis 27,5 g/m² liegt, und Transpirationsfähigkeitswerte aufweist, die in einem Bereich von ungefähr 1300 bis 1700 g/m²/24h liegen.

2. Artikel nach Anspruch 1, **dadurch gekennzeichnet, dass** die Folie ein Gramm-Oberflächen-Verhältnis aufweist, das in einem Bereich von 22 bis 26 g/m² liegt.

3. Artikel nach Anspruch 1 oder 2, **dadurch gekennzeichnet, dass** das Gramm-Oberflächen-Verhältnis in einem Bereich von 24 bis 25,5 g/m² liegt.

4. Artikel nach einem der Ansprüche 1 bis 3, **dadurch gekennzeichnet, dass** die Folie eine Reißfestigkeit in Maschinenrichtung im Bereich von 27 bis 29 N/Zoll und eine Bruchdehnung in Maschinenrichtung im Bereich von 210 bis 250 N/Zoll aufweist.

5. Artikel nach einem der Ansprüche 1 bis 4, **dadurch gekennzeichnet, dass** die Folie eine Reißfestigkeit in Maschinenquerrichtung von 11 bis 12 N/Zoll und eine Bruchdehnung in Maschinenquerrichtung von 650 bis 750 N/Zoll aufweist.

6. Artikel nach einem der Ansprüche 1 bis 5, **dadurch gekennzeichnet, dass** das biologisch abbaubare thermoplastische Material Mater-Bi ist.

7. Artikel nach einem der Ansprüche 1 bis 6, **dadurch gekennzeichnet, dass** dieser aufgrund des Vorhandenseins von biologisch abbaubaren natürlichen Pigmenten, die ihm ein äußeres Aussehen geben, das ähnlich ist wie das eines Kleidungsstückes, undurchsichtig ist.

8. Artikel nach Anspruch 6 oder 7, **dadurch gekennzeichnet, dass** dieser auch ein Sperrensystem umfasst, um das Auslaufen von Fäkalien und Flüssigkeiten beim Tragen wesentlich zu begrenzen.

9. Artikel nach einem der Ansprüche 6 bis 8, **dadurch gekennzeichnet, dass** dieser auch ein Verschlusssystem, das an äußeren Laschen angeordnet ist, sowie elastisch eingerichtete Fäkaliensperren nahe bei den beiden seitlichen Schenkelöffnungen umfasst.

10. Artikel nach einem der Ansprüche 6 bis 9, **dadurch gekennzeichnet, dass** die zentrale absorbierende Schicht ein biologisch abbaubares absorbierendes Material umfasst.

11. Artikel nach Anspruch 10, **dadurch gekennzeichnet, dass** das biologisch abbaubare absorbierende Material Zelluloseflaum, Baumwolle, natürliche absorbierende Fasern und deren Mischungen umfasst.

12. Artikel nach einem der Ansprüche 6 bis 11, **dadurch gekennzeichnet, dass** dieser auch eine oder mehrere Schichten umfasst, die für Flüssigkeiten durchlässig sind und die über der zentralen absorbierenden Schicht angeordnet sind.

13. Artikel nach einem der Ansprüche 6 bis 12, **dadurch gekennzeichnet, dass** elastische Fäden auf die Innenfläche der äußeren Schicht und nahe bei den Schenkelöffnungen aufgebracht sind, um das Auslaufen von Körperflüssigkeiten zu verhindern.

14. Artikel nach einem der Ansprüche 6 bis 13, **dadurch gekennzeichnet, dass** die äußere Schicht, die für Flüssigkeiten undurchlässig ist, biologisch abbaubare natürliche Pigmente oder Mater-Batch umfasst, um den Artikeln ein Aussehen zu verleihen, das ähnlich ist wie das eines Kleidungsstückes.

15. Artikel nach einem der Ansprüche 1 bis 14, **dadurch gekennzeichnet, dass** dieser eine Windel ist.

## Revendications

1. Article hygiénique et biodégradable absorbant comprenant un film biodégradable réalisé dans un matériau thermoplastique biodégradable, convenant pour une couche transpirante destinée à contenir des fluides organiques, **caractérisé en ce que** ledit film est microporeux en raison de la présence d'une série de micropores qui passent à travers celui-ci en permettant la transpiration, **en ce qu'**il est imperméable aux liquides, et **en ce qu'**il a un rapport gramme-surface allant de 18 à 27,5 g/m² et des valeurs d'aptitude à la transpiration allant de 1300 à 1700 g/sm/24 h environ.

2. Article selon la revendication 1, **caractérisé en ce que** ledit film a un rapport gramme-surface allant de 22 à 26 g/m².

3. Article selon la revendication 1 ou 2, **caractérisé en ce que** ledit rapport gramme-surface va de 24 à 25,5 g/m².

4. Article selon l'une quelconque des revendications précédentes 1 à 3, **caractérisé en ce que** ledit film a une résistance à la traction maximale dans le sens machine allant de 27 à 29 N/pouce et un allongement maximal dans le sens machine allant de 210 à 250 N/pouce.

5. Article selon l'une quelconque des revendications précédentes 1 à 4, **caractérisé en ce que** ledit film a une résistance à la traction maximale dans le sens travers allant de 11 à 12 N/pouce et un allongement maximal dans le sens travers allant de 650 à 750 N/pouce.

6. Article selon l'une quelconque des revendications précédentes 1 à 5, **caractérisé en ce que** ledit matériau thermoplastique biodégradable est du Mater-Bi.

7. Article selon l'une quelconque des revendications précédentes 1 à 6, **caractérisé en ce qu'**il est opaque en raison de la présence de pigments naturels biodégradables qui lui donnent une apparence esthétique similaire à celle d'un vêtement.

8. Article selon la revendication 6 ou 7, **caractérisé en ce qu'**il comprend également un système formant barrière destiné à limiter sensiblement les fuites de matières fécales et de liquides lorsqu'il est porté.

9. Article selon l'une quelconque des revendications 6 à 8, **caractérisé en ce qu'**il comprend également un système de fermeture positionné sur des rabats extérieurs, et des barrières élastiques pour les matières fécales situées à proximité de deux ouvertures latérales pour les jambes.

10. Article selon l'une quelconque des revendications 6 à 9, **caractérisé en ce que** ladite couche centrale absorbante comprend un matériau absorbant biodégradable.

11. Article selon la revendication 10, **caractérisé en ce que** ledit matériau absorbant biodégradable comprend du duvet de cellulose, du coton, des fibres naturelles absorbantes et leurs mélanges.

12. Article selon l'une quelconque des revendications 6 à 11, **caractérisé en ce qu'**il comprend également une ou plusieurs couches perméables aux liquides disposées au-dessus de ladite couche centrale absorbante.

13. Article selon l'une quelconque des revendications 6 à 12, **caractérisé en ce que** des fils élastiques sont appliqués sur la surface intérieure de ladite couche extérieure et à proximité des ouvertures pour les jambes de manière à empêcher les fuites de liquides organiques.

14. Article selon l'une quelconque des revendications 6 à 13, **caractérisé en ce que** ladite couche extérieure imperméable aux liquides comprend des pigments naturels biodégradables ou du Mater Batch de manière à donner auxdits articles une apparence similaire à celle d'un vêtement.

15. Article selon l'une quelconque des revendications 1 à 14, **caractérisé en ce qu'**il est une couche.
